# EUROPEAN PATENT SPECIFICATION

(11) **EP 0 910 620 B1**
(45) Date of publication and mention of the grant of the patent: **28.08.2002**
(21) Application number: 97923033.1
(22) Date of filing: 02.05.1997
(51) Int. Cl.: C12C 7/04, C12C 5/02, C12P 7/06

(54) **METHOD FOR MAKING WORT HAVING IMPROVED FILTERABILITY AND/OR INCREASED YIELD**
VERFAHREN ZUR HERSTELLUNG VON WÜRZE MIT VERBESSERTER FILTRIERBARKEIT UND/ODER ERHÖHTER AUSBEUTE
PROCEDE DE PRODUCTION DE MOUT A FILTRABILITE ET/OU RENDEMENT AMELIORES

(30) Priority: 03.05.1996 EP 96201197; 30.12.1996 EP 96203620
(43) Date of publication of application: 28.04.1999
(73) Proprietor: DSM N.V., 6411 TE Heerlen (NL)
(72) Inventor: LAROYE, Marie-Paule, F-59113 Seclin (FR); SOUPPE, Jerome, F-59290 Wasquehal (FR)
(74) Representative: Visser-Luirink, Gesina, Dr.
(86) International application number: EP9702372
(87) International publication number: WO97042301

(56) References cited:
- WO-A-95/23514
- WO-A-96/06935
- US-A- 5 372 939
- US-A- 5 487 989
- CEREAL CHEMISTRY, vol. 72, no. 4, July 1995 - August 1995, MINNEAPOLIS US, pages 360-364, XP000519374 THOMAS, K.C. ET AL.: "Production of fuel alcohol from hull-less barley by very high gravity technology."

## Description

### Field of the invention

The present invention provides a process for making wort having improved filterability and/or showing increased brewing yield. The invention also provides for methods of brewing beer, making wine and potable alcohol from wort made according to the present invention. The invention also concerns the use of enzymes in improving filterability and/or yield of wort made from malted and/or unmalted cereals, as well as compositions comprising mixtures of enzyme activities according to the invention.

### Background of the invention

The use of enzymes in making fermentable wort is known for a long time. For example, for brewing beer grains and/or malted grains are liquefied and saccharified in order to yield fermentable sugars. Liquefaction steps may be improved by the use of thermostable α-amylases as described for instance in US 4,285,975 or US 5,180,669. Also proteases are used to increase the amount of freely available nitrogen in the wort to improve fermentation. Alter filtration of the liquefied and saccharified mash, the obtained wort is inoculated with special strains of yeast (*Saccharomyces* sp.) which convert sugars into ethanol and characteristic flavour compounds.

However, apart from starch other polysaccharides are present in cereal grains. For example β-glucans are present (R.J. HENRY J.Sci Food Agric. (1985) 36, 1243-1253) as listed below for various cereals:

| Cereal | % β-glucans |
|---|---|
| wheat | 0.5 - 8.0 |
| barley | 4.0 - 8.0 |
| rye | 5.0 - 10.0 |
| rice | 1.0 - 3.0 |
| oat | 5.0 - 10.0 |

These β-glucans consist in β-1,3/β-1,4 linkages between β-D-glucopyranose moieties (M.J.EDNEY, B.A. MARCHYLO and A.W. Mac GREGOR J. Inst. Brew. (1991) 97, 39-44). β-glucans are highly viscous (N.WAGNER *et al.* Monatschrift für Brauwissenschaft (1988) Heft **10,** 384-395) and bring wort and beer filtration problems (S.AASTRUP Carlsberg Res. Commun. (1979) **44**, 289-304) if they are not hydrolysed during the liquefaction step. This is the reason why β-glucanases from *Bacillus subtilis* (R.BORRISS Zeitschr. für allgem. Mikrobiol. (1981) **21**(1), 7-17), *Penicillium emersonii* (A.P.MOLONEY, *et al*. Enzym. Microb. Technol. (1983) **5,** 260-264), *Mucor miehei* (F.BRANISLAV European Patent 0 504 947 A2, 1992) or *Trichoderma* sp. (S.P. SHOEMAKER and R.D.BROWN Jr Biochim. Biophys. Acta (1978) **523**, 133-146) are widely used at industrial scale, in addition to the β-glucanase present in the malt; the latter is not sufficiently thermostable to be active during the brewing diagramme processing (M.HRMOVA and G.B. FINCHER Biochem. J. (1993) **289**, 453-461).

Non-starch polysaccharides also include pentosans, the structure of which have been widely studied recently (H.GRUPPEN *et al.* Carbohydr. Res. (1992) **233**, 45-64; G.ANNISON *et al*., Carbohydr. Polym. (1992) **19**, 151-159; T. ITO *et al*., J. Carbohydr. Chem. (1994) **13**(3) 491-498) in particular those of barley and malt (R.J. VIETOR *et al*., Carbohydr. Res. (1994) **254**, 245-255; R.J. VIETOR *et al*., Carbohydr. Polym. (1994) **24**, 113-118). British patent 2,150,933 shows the interest for a pentosanase from *Penicillium emersonii* to improve the production and extraction of fermentable sugars in brewing. US Patent 4,746,517 demonstrates the high efficiency of the xylanolytic system from *Disporotrichum dimorphosporum* to improve wort and beer filterability.

The use of xylanase B to improve filterability of wort has also been mentioned in WO94/14965.

Applications which may be mentioned in conjunction with the use of enzymes in making wort for fermentation are:
WO94/21785.

Despite the advance which has been made in this area, there is still a need for methods of preparing wort with further improved filterability and/or higher yields, and enzyme preparations for use therein.

### Summary of the invention

The present invention provides worts with increased yield and improved filterability as well as a process for preparing said wort comprising the steps of:
(a) preparing mash from malted or unmalted cereals, or a mixture of malted and unmalted cereals, in the presence of a mixture of enzyme activities,
(b) filtering the mash so obtained to obtain the wort,
wherein said mixture of enzyme activities is selected from a mixture comprising at least β-glucanase activity and α-L-arabinofuranose releasing activity or a mixture comprising at least β-glucanase activity endo-xylanase activity and α-L-arabinofuranose releasing activity. Preferably, according to the process of the invention, α-L-arabinofuranose releasing activity is provided by an enzyme selected from α-L-arabinofuranosidase (EC 3.2.1.55) and (1->4)-β-D-arabinoxylan arabinofuranohydrolase (AXH), or a mixture of the said enzymes. The use of α-L-arabinofuranosidase or AXH obtainable from *Aspergillus* strain is still further preferred. According to one embodiment α-L-arabinofuranosidase A from *Aspergillus niger* is used.

According to another embodiment the invention provides a process for making beer, and/or potable alcohol, and other alcoholic beverages, wherein a wort is fermented obtainable according to the invention.

According to a further embodiment the invention envisages the use of α-L-arabinofuranosidase B from *Aspergillus niger* in a process of improving filterability of wort.

According to another embodiment the invention provides for the use of α-L-arabinofuranosidase A in a process of increasing yield of wort.

The invention also provides an enzyme preparation suitable for use in a process of making wort, said composition comprising a mixture of enzyme acitivities selected from a mixture comprising at least β-glucanase activity and α-L-arabinofuranose releasing activity or a mixture comprising at least β-glucanase activity endo-xylanase activity and α-L-arabinofuranose releasing activity.

Preferred according to the invention is an enzyme preparation, wherein said α-L-arabinofuranose releasing activity is provided for by an enzyme selected from α-L-arabinofuranosidase (EC 3.2.1.55) and AXH, or a mixture thereof. Preferred according to the invention is an enzyme preparation wherein said α-L-arabinofuranosidase or said AXH is obtainable from *Aspergillus* strain.

### Description of the invention

We have now surprisingly found that when wort is made in the presence of a mixture of enzyme activities comprising β-glucanase activity, α-L-arabinofuranosidase activity and preferably endo-β-1,4--xylanase, a liquefied and saccharified mash is obtained which can be filtered much easier than mash obtained using β-glucanases alone or mixtures of β-glucanases and endo-xylanase. Moreover, the wort obtained after filtration of the mash shows higher yield. Yield in this regard refers to the amount of fermentable sugars in the wort, expressed as a percentage of sugars present in the raw materials. The yield improvement of the wort is dependent on the malt chosen. The malts exemplified herein show already good yields without the enzymes activities according to the invention. It is envisaged that more dramatic improvements may be obtained with poorer quality malts, with unmalted cereals or with mixed brews.

The manufacturing of the wort may be performed conventionally, involving liquefaction and saccharification of cereal material, usually with the aid of α-amylases and proteases, to obtain a liquefied and saccharified mash.

Suitable starting materials are cereals, either so-called raw or unmalted material or malted, or mixtures thereof (mixed brew). For example, cereals such as barley, wheat, corn, sorghum, oat and rice can be used, either malted or raw, or mixed. Preferably, the method according to the invention is used in 100% malt brew.

In the case of raw cereals, the liquefaction step usually comprises grinding of the cereal raw material to obtain a flour of suitable particle size, hydrating with from about 1 to about 4, preferably about 3 parts of water, and optionally, depending on the endoprotease used, from about 50 to about 300 ppm of calcium, preferably 200 ppm Ca²⁺. Enzymes from *Bacillus stearothermophilus* appear to be less Calcium-dependent. Consequently, no Ca²⁺ supplementation is required in that case. The particle size of the ground cereals should not exceed about 3 mm; not more than 3,5% should exceed 1,3 mm; not more than 1,5% should be smaller than 0.25 mm. Enzymes that may be used in addition are cellulases. β-glucanases, and or other plant cell wall degrading enzymes.

The liquefaction medium is usually adjusted to a pH of between about 5 and 8, preferably between about 6 and 7, using, for example, calcium hydroxide. It is important to add α-amylase, preferably a thermostable α-amylase to the liquefaction medium as well as an endoprotease in a dosage sufficient to at least partially liquefy the cereal starch, and to at least partially degrade protein. Suitable dosages of α-amylase are from about 0,5 to about 2,0, preferably about 1 - 1,5 kg per Ton, when B.A.T.S. is used. Suitable dosages of proteases are, in the case of Brewers protease 2000, more than 0.5 kg/Ton grains (kg/T), preferably more than 1 kg/T. In the case of Panstimase 400 more than 2 kg/T, preferably more than 5, more preferably more than 10 kg/T should be used.

In the liquefaction process a number of steps are usually carried out at elevated temperature: after adding α-amylase and protease the mixture is maintained at a temperature between about 40°C and 65°C, preferably between about 45 and 55°C, most preferably 50°C, until a sufficient liquefaction is obtained. The time needed depends on the cereal or mixture of cereals used, but usually from about 30 minutes till about 2 hours is satisfactory. Subsequently, the temperature is raised gradually, the rate not being critical, till about 90-95°C and left at that temperature for about 30 minutes to about 1 hour. Then, the mixture is cooled to a temperature at which saccharification takes place: usually at about 50°C to about 70°C, preferably between about *55°C* and 65°C, most preferably about 60°C. Slightly higher temperatures than 70°C should be possible, depending on the thermostability of the enzymes used in the saccharification step. When the preferred temperature is reached saccharifying enzymes are added, such as Brewers fermex (α-amylase) or Novamyl (recombinant β-amylase) in amounts usually ranging from about 400 g/T to about 1 kg/T for Brewers fermex. Also glucoamylases are frequently used. The saccharification takes from about 30 minutes to about 2 hours, whereafter the temperature is raised to about 75°C to about 85°C, *inter alia* to inactivate enzymes and unwanted microorganisms, and kept at the preferred elevated temperature for about 10 minutes; the period is not very critical.

The mash so obtained is subsequently filtered using equipment well known in the art; a funnel with Schleicher & Schuell paper filter works satisfactorily. After filtration, the wort is fermented by a suitable yeast, under conditions depending on the strain used, and the final purpose; in addition to brewing beer, production of alcohol as biofuel or as alcoholic beverage are envisaged by the instant invention. Suitable strains, and suitable conditions are well known to the person skilled in the art.

Other enzymes in the prepration of the wort that are conventionally used are β-glucanases. The β-glucanase is preferably thermostable enough to be active during the first step of a standard brewing diagramme (typically several minutes at 50°C and pH 5.6). Several microbial enzymes can fullfil this requirement e.g. β-glucanase from *Penicillium emersonii, Trichoderma longibrachiatum, Bacillus amyloliquefaciens*. Excellent results may be obtained with β-glucanase from *Bacillus amyloliquefaciens* commercially available from Gist-Brocades under the trademark Filtrase L (+), having an activity of 600 BGR/g. Preferably, β-glucanase is obtained from a recombinant strain of *Bacillus amyloliquefaciens*. Usually also proteases may be used, such as Brewer's Protease and the like.

Endo-xylanases that may be used have been described in the section background art.

Endo-β-1,4-xylanase (preferably isoenzyme endo I (R.A. FOURNIER Biotechnology and Bioeng.(1985) **XXVII**, 539-546) and α-L-arabino-furanosidase (preferably isoenzyme A (F.J.M. KORMELINK *et al*., Carborhydr. Res. (1993) **24**, 345-353)) may be obtained from wild-type, mutated or recombinant strains of *Aspergillus niger.*

As regards the α-L-arabinofuranose releasing activities, several enzymes may be envisaged. We have shown that AXH leads to better filterability. Two α-L-arabinosidases from *Aspergillus niger* appeared to lead to significantly higher filtration rates. As the mode of action of AXH, α-L-arabinosidase A and B all differ, it may be expected, that a mixture of the three α-L-arabinofuranose releasing activities may yield to still better filtration rates.

Using the process according to the invention substantially improved filtration rates may be obtained of the liquefied and saccharified mash. This brings advantages in terms of a speedier process, less clogging of filters, and larger wort volumes.
Also the improved yield leads to a more economic brewing process. The wort according to the invention can be used in brewing beer, or making potable alcohol or biofuel, or in a process of making other alcoholic beverages. The practicing of the invention and the associated advantages are illustrated in greater detail in the following non-limitative Examples.

### Experimental part

### 1/β-glucanase

In the Examples β-glucanase was used from *Bacillus amyloliquefaciens* commercially available from Gist-Brocades under the trademark Filtrase L (+), having an activity of 600 BGR/g.

### 2/Endo-β-1,4-xylanase

Endoxylanase was obtained from a pure culture of *Aspergillus niger* in a sterile tank and medium. The culture medium contains appropriate carbon and nitrogen sources just as mineral salts. The fermentation is carried out at a constant temperature between 30-40°C and pH is maintained within the range 3-5. The activity of the enzyme is measured by the hydrolysis of xylan from oat spelts suspended (35g/l) in 1M glycine buffer pH 2,75. the viscosity of this solution is determined by using a capillary viscometer (Ubbelhode type) at 47°C. The time dt needed for the upper menisk of the liquid to fall down between two reference points is measured within time T. The slope of the plot T versus 1/dt yields an apparent kinetic constant. 1 Lyx unit is the amount of enzyme needed to reach a value of 1 min-1 for that kinetic constant.

### 3/α-L-arabinofuranosidase

Isoenzyme A or isoenzyme B (F.J.M. KORMELINK *et al*., Carborhydr. Res. (1993) **24**, 345-353) or AXH (F.J.M. KORMELINK *et al*., Appl. Microbiol. Biotechnol. (1991) **35**, 753-758) have been obtained from a culture of recombinant *Aspergillus niger* or *Aspergillus nidulans* strains. The amino acid sequences, their encoding DNA, as well as the recombinant production of α-L-arabinofuranosidase A and B is described in detail in European patent application 0 506 190 A1, published on September 30, 1992, particularly in the Examples and Figures and the Sequence Listing, which relevant parts are herein incorporated by reference.
Activity of isoenzymes A and B is measured by the hydrolysis of p-nitrophenyl-α-L-arabinofuranoside. 1 ARF unit is the amount of enzyme needed to liberate 1 µmole p-nitrophenol per minute under the conditions of the test described in (Z. GUNATA et al., J. Agric. Food Chem. (1989) **38**, 772).

### Example 1

Wort was prepared from malt ground according to standard specifications for lauter tun filtration. Malt was ground with the EBC MIAG mill according to standard specifications, i.e. yielding a difference in fine (1 mm) to coarse (2.5 mm) extract in the range 1.5-2 %. One part malt is hydrated with 3 parts water or aqueous solution of enzyme at 50°C. This temperature is maintained during 20 minutes; it is then raised up to 63°C (1°C/min) and maintained at that temperature during 30 minutes. The medium is then heated at 72°C (1°C/min) and maintained at this temperature during 20 minutes. It is finally heated at 76°C and maintained at that temperature for 5 minutes. Water is added to compensate for water evaporation.

The mash is poured into a funnel containing Schleicher and Schuell paper filter. The volume of filtered wort is measured after 15 minutes. Specific gravity is determined at the end of the filtration. This value allows to calculate extract and yield.

### 1st serie (British malt).

5 brews have been carried out with different enzymes as shown in Table 1 :

| Brew n° | Enzymatic units/kg malt | | | | |
|---|---|---|---|---|---|
| | BGR | LYX | ARF-A | AXH | ARF-B |
| 1 | 0 | 0 | 0 | 0 | 0 |
| 2 | 90 | 1200 | 0 | 0 | 0 |
| 3 | 90 | 1200 | 1500 | 0 | 0 |
| 4 | 90 | 1200 | - | 0 | 1500 |
| 5 | 90 | 1200 | 0 | 1500 | 0 |

In brew n°3, ARF is from α-L-arabinofuranosidase isoenzyme A
In brew n°4, ARF is from α-L-arabinofuranosidase isoenzyme B
In brew n°5, AXH (F.J.M. KORMELINK *et al.* Appl. Microbiol. Biotechnol. (1991) 35, 753-758) is used to hydrolyse the α-L-arabinofuranose moieties of arabinoxylans. The dose was 0,15 mg pure AXH per kg malt (AXH is not active on p-nitrophenyl-α-L-arabinofuranoside, therefore AXH has no ARF activity).

Results are presented in Table 2:

| Brew n° | Volume filtered after 15 minutes (ml) | Yield (%) |
|---|---|---|
| 1 | 62 | 83,04 |
| 2 | 88 | 83,69 |
| 3 | 102 | 83,64 |
| 4 | 106 | 83,24 |
| 5 | 100 | 83,44 |

### 2nd serie (French malt)

The same brews as described in the 1st series have been produced from a french malt. Results are presented in Table 3 :

| Brew n° | Volume filtered after 15 minutes (ml) | Yield (%) |
|---|---|---|
| 1 | 54 | 82,35 |
| 2 | 82 | 82,50 |
| 3 | 88 | 83,71 |
| 4 | 92 | 82,60 |
| 5 | 88 | 82,50 |

From these 2 series, the combination β-glucanase + endoxylanase + α-L-arabinofuranosidase isoenzyme B performs best to improve wort filtration, whereas the same combination in which α-L-arabino-furanosidase isoenzyme A replaces α-L-arabinofuranosidase isoenzyme B performs best to improve the yield, the filtration being also highly improved in comparison with the blank (brew 1).

### Example 2

Worts were produced the same way as in example 1, always with the same batch of malt but varying the composition of the enzymes mixture. 12 brews were carried out according to an experimental design in order to determine the role of each component of the mixture with regards to yield and filtration improvement. In all trials, α-L-arabinofuranosidase was isoenzyme A from *A. niger.*

All runs performed are presented in Table 4 :

| Brew n° | Enzymatic units/kg malt | | |
|---|---|---|---|
| | BGR | LYX | ARF (isoenzyme A) |
| 1 | 30 | 300 | 300 |
| 2 | 90 | 300 | 300 |
| 3 | 30 | 1200 | 300 |
| 4 | 90 | 1200 | 300 |
| 5 | 30 | 300 | 1500 |
| 6 | 90 | 300 | 1500 |
| 7 | 30 | 1200 | 1500 |
| 8 | 90 | 1200 | 1500 |
| 9 | 60 | 750 | 900 |
| 10 | 60 | 750 | 900 |
| 11 | 60 | 750 | 900 |
| 12 | 0 | 0 | 0 |

Results are shown in Table 5 :

| Brew n° | Volume filtered after 15 minutes (ml) | Yield (%) |
|---|---|---|
| 1 | 115 | 80,16 |
| 2 | 100 | 80,21 |
| 3 | 116 | 80,01 |
| 4 | 110 | 80.81 |
| 5 | 116 | 80,11 |
| 6 | 128 | 80,26 |
| 7 | 122 | 80,01 |
| 8 | 142 | 80,56 |
| 9 | 116 | 80,06 |
| 10 | 120 | 80,11 |
| 11 | 117 | 80,11 |
| 12 | 89 | 80,01 |

From Brews n°9-10-11 standard deviations may be determined :
2,1 ml for volume filtered after 15 minutes
0,03% for the yield

From Brews n° 1 to 8, the effect of each component just as those of interactions between components may be determined:

| Cause | Effect on | |
|---|---|---|
| | Filterability | Yield |
| BGR | +2,75 | +0,39 |
| LYX | +7,75 | +0,16 |
| ARF | +16,75 | -0,06 |
| BGR*LYX | +4,25 | +0,29 |
| BGR*ARF | +13,25 | -0,04 |
| LYX*ARF | +2,75 | -0,06 |

Only figures in bold type characters may be considered as significant (> 95%). α-L-arabinofuranosidase A alone and in combination with β-glucanase brings the strongest effect in filtration improvement. whereas β-glucanase, endoxylanase alone and in combinations contribute significantly to the yield improvement.

### Example 3

In that serie, the same combination of enzymes is used :
60 BGR/kg malt
+ 750 LYX/kg malt
+ 900 ARF/kg malt
but different malt were brewed with and without this enzyme combination. Worts were produced according to the same procedure as in Example 1.

Results are presented in Table 5 :

| Malt's origin | Volume filtered after 15 minutes (ml) | | Yield (%) | |
|---|---|---|---|---|
| | without enzyme | with enzyme | without enzyme | with enzyme |
| USA (1) | 131 | 171 | 79,75 | 79,77 |
| USA (2) | 114 | 164 | 83,74 | 83,97 |
| UK | 117 | 167 | 81,08 | 83,21 |
| Canada (1) | 106 | 153 | 81,42 | 81,52 |
| Canada (2) | 99 | 137 | 81,30 | 81,86 |
| France | 89 | 118 | 80,18 | 80,09 |

Thus, the mixture of enzyme activities is efficient for filtration improvement, whatever the malt brewed. The effect on yield is low but this is mainly because the malts used are already of a very high quality.

### Example 4

Wheat is used as crude adjunct in several breweries. This cereal contains a high amount of pentosans. This is one reason to test the above mentioned combination of enzymes in a mixed brew.

Worts were produced according to the same procedure as in example 1 but 20% malt was replaced by 20% wheat.

Table 6 describes all runs performed :

| Brew n° | Enzymatic units/kg malt | | |
|---|---|---|---|
| | BGR | LYX | ARF |
| 1 | 0 | 0 | 0 |
| 2 | 90 | 0 | 0 |
| 3 | 0 | 120 | 0 |
| 4 | 0 | 120 | 300 |
| 5 | 0 | 120 | 1500 |
| 6 | 90 | 120 | 1500 |

Results are given in Table 7

| Brew n° | Volume filtered after 15 minutes (ml) | Yield (%) |
|---|---|---|
| 1 | 66 | 81,01 |
| 2 | 89 | 81,52 |
| 3 | 62 | 80,81 |
| 4 | 69 | 80,91 |
| 5 | 80 | 80,91 |
| 6 | 99 | 81,52 |

These results confirm the interest for the combination described in the invention in the case of brews involving wheat adjuncts.

### Example 5

In this example, wort was prepared from crude barley grains, variety PLAISANT. Barley grains were ground with the EBC MIAG mill in order to make filter press type barley flour. 57g barley flour are added in 300ml water or aqueous solution of enzymes at 50°C. This temperature is maintained for 1h; it is then heated up to 63°C (1°C/min) and maintained at that temperature during 30 minutes. The medium is then heated up to 90°C (1°C/min) and maintained at that temperature during 20 minutes. Water is added to compensate for water evaporation. The mash is then poured into a funnel containing Schleicher and Schuell paper filter.

6 brews have been carried out with different enzymes as shown in Table 8 (ARF when mentioned is from **Ara A**):

| Brew No. | Enzymes | |
|---|---|---|
| | Microbial origin | Units/kg barley |
| 1 | - | 0 |
| 2(*) | *Bacillus amyloliquefaciens* | 90 BGR |
| | *+ Aspergillus niger* | + 1200 Lyx + 1500 ARF |
| 3(*) | idem | 90 BGR |
| | | + 12000 Lyx + 15000 ARF |
| 4 | *Penicillium emersonii* (**) | 36 XVU |
| 5 | *Bacillus amyloliquefaciens* | 90 BGR |
| | + *Disporotrichum dimorphosporum* (***) | + 120 XVU |
| 6 | *Trichoderma viride* (****) | 36 XVU |

| | | |
|---|---|---|
| (*) according to the invention | | |
| (**) commercially available from Gist-brocades under the trade name Filtrase® NL | | |
| (***) commercially available from Gist-brocades under the trade name Filtrase® Br | | |
| (****) commercially available from Gist-brocades under the trade name Natugrain® | | |

Results are presented in Table 9.

| Brew No. | Volume (ml) filtered after 15 minutes |
|---|---|
| 1 | 111 |
| 2 | 117 |
| 3 | 164 |
| 4 | 141 |
| 5 | 172 |
| 6 | 136 |

These results show that the combination described in the invention is at least as good or even better performing than existing preparations in the case of brews involving 100% unmalted barley.

## Claims

1. A process for preparing wort comprising the steps of:
(a) preparing mash from malted or unmalted cereals, or a mixture of malted and unmalted cereals, in the presence of a mixture of enzyme activities,
(b) filtering the mash to obtain the wort,
wherein said mixture of enzyme activities is selected from a mixture comprising at least β-glucanase activity and α-L-arabinofuranose releasing activity or a mixture comprising at least β-glucanase activity endoxylanase activity and α-L-arabinofuranose releasing activity.

2. A process according to claim 1, wherein said α-L-arabinofuranose releasing activity is provided by an enzyme selected from α-L-arabinofuranosidase (EC 3.2.1.55) and (1->4)-β-D-arabinoxylan arabinofuranohydrolase (AXH), or a mixture of the said enzymes.

3. A process according to claim 1, wherein said α-L-arabinofuranosidase or said (1->4)-β-D-arabinoxylan arabinofuranohydrolase (AXH) is obtainable from an *Aspergillus* strain.

4. A process according to claim 3, wherein said α-L-arabinofuranosidase is α-L-arabinofuranosidase A from *Aspergillus niger.*

5. A process according to claim 3, wherein said α-L-arabinofuranosidase is α-L-arabinofuranosidase B from *Aspergillus niger.*

6. A process according to claim 1-5, wherein the wort is further fermented to produce beer and/or potable alcohol.

7. Use of α-L-arabinofuranosidase B from *Aspergillus niger* in a process of improving filterability of wort.

8. Use of α-L-arabinofuranosidase A in a process of increasing yield of wort.

9. An enzyme preparation suitable for use in a process of making wort, said composition comprising a mixture of enzyme acitivities selected from a mixture comprising at least β-glucanase activity and α-L-arabinofuranose releasing activity or a mixture comprising at least β-glucanase activity endoxylanase activity and α-L-arabinofuranose releasing activity.

10. An enzyme preparation according to claim 9, wherein said α-L-arabinofuranose releasing activity is provided for by an enzyme selected from α-L-arabinofuranosidase (EC 3.2.1.55) and (1->4)-β-D-arabinoxylan arabinofuranohydrolase (AXH), or a mixture thereof

11. An enzyme preparation according to claim 10, wherein said α-L-arabinofuranosidase or said (1->4)-β-D-arabinoxylan arabinofuranohydrolase (AXH) is obtainable from an *Aspergillus* strain.

## Patentansprüche

1. Verfahren zur Herstellung von Würze, umfassend die Schritte:
(a) Herstellen einer Maische aus vermälztem oder unvermälztem Getreide oder einem Gemisch aus vermälztem oder unvermälztem Getreide in Gegenwart eines Gemisches aus Enzymaktivitäten,
(b) Filtrieren der Maische, um die Würze zu erhalten,
wobei besagtes Gemisch aus Enzymaktivitäten ausgewählt wird aus einem Gemisch, welches mindestens β-Glucanaseaktivität und α-L-Arabinofuranose-freisetzende Aktivität umfasst, oder einem Gemisch, welches mindestens β-Glucanaseaktivität Endoxylanaseaktivität und α-L-Arabinofuranose-freisetzende Aktivität umfasst.

2. Verfahren gemäß Anspruch 1, wobei besagte α-L-Arabinofuranose-freisetzende Aktivität durch ein Enzym vorgesehen wird, welches ausgewählt wird aus α-L-Arabinofuranosidase (EC 3.2.1.55) und (1->4)-β-D-Arabinoxylan-arabinofuranohydrolase (AXH) oder einem Gemisch der besagten Enzyme.

3. Verfahren gemäß Anspruch 1, wobei besagte α-L-Arabinofuranosidase oder besagte (1->4)-β-D-Arabinoxylan-arabinofuranohydrolase (AXH) von einem *Aspergillus*-Stamm erhältlich ist.

4. Verfahren gemäß Anspruch 3, wobei besagte α-L-Arabinofuranosidase α-L-Arabinofuranosidase A von *Aspergillus niger* ist.

5. Verfahren gemäß Anspruch 3, wobei besagte α-L-Arabinofuranosidase α-L-Arabinofuranosidase B von *Aspergillus niger* ist.

6. Verfahren gemäß Ansprüchen 1-5, wobei die Würze weiter fermentiert wird, um Bier und/oder trinkbaren Alkohol herzustellen.

7. Verwendung von α-L-Arabinofuranosidase B von *Aspergillus niger* in einem Verfahren zum Verbessern der Filtrierbarkeit von Würze.

8. Verwendung von α-L-Arabinofuranosidase A in einem Verfahren zum Steigern der Ausbeute von Würze.

9. Enzympräparat, welches zur Verwendung in einem Verfahren zum Herstellen von Würze geeignet ist, wobei besagte Zusammensetzung ein Gemisch aus Enzymaktivitäten umfasst, ausgewählt aus einem Gemisch, welches mindestens β-Glucanaseaktivität und α-L-Arabinofuranose-freisetzende Aktivität umfasst, oder einem Gemisch, welches mindestens β-Glucanaseaktivität Endoxylanaseaktivität und α-L-Arabinofuranose-freisetzende Aktivität umfasst.

10. Enzympräparat gemäß Anspruch 9, worin besagte α-L-Arabinofuranose-freisetzende Aktivität durch ein Enzym vorgesehen wird, welches ausgewählt wird aus α-L-Arabinofuranosidase (EC 3.2.1.55) und (1->4)-β-Arabinoxylan-arabinofuranohydrolase (AXH) oder einem Gemisch davon.

11. Enzympräparat gemäß Anspruch 10, worin besagte α-L-Arabinofuranosidase oder besagte (1->4)-β-D-Arabinoxylan-arabinofuranohydrolase (AXH) von einem *Aspergillus*-Stamm erhältlich ist.

## Revendications

1. Procédé de préparation d'un moût comprenant les étapes consistant :
(a) à préparer une maische à partir de céréales maltées ou non maltées, ou d'un mélange de céréales maltées ou non maltées, en présence d'un mélange d'activités enzymatiques, et
(b) à filtrer la maische ainsi obtenue pour obtenir le moût,
dans lequel ledit mélange d'activités enzymatiques est choisi parmi un mélange comprenant au moins une activité de β-glucanase et une activité de libération d'α-L-arabinofuranose ou un mélange comprenant au moins une activité de β-glucanase, une activité d'endo-xylanase et une activité de libération d'α-L-arabinofuranose.

2. Procédé selon la revendication 1, dans lequel ladite activité de libération d'α-L-arabinofuranose est fournie par une enzyme choisie parmi l'α-L-arabinofuranosidase (EC 3.2.1.55) et la (1->4)-β-D-arabinoxylan-arabinofuranohydrolase (AXH), ou un mélange desdites enzymes.

3. Procédé selon la revendication 1, dans lequel ladite α-L-arabinofuranosidase ou ladite (1->)-β-D-arabinoxylan-arabinofuranohydrolase (AXH) peut être obtenue à partir d'une souche de *Aspergillus.*

4. Procédé selon la revendication 3, dans lequel ladite α-L-arabinofuranosidase est l'α-L-arabinofuranosidase A provenant de *Aspergillus niger.*

5. Procédé selon la revendication 3, dans lequel ladite α-L-arabinofuranosidase est l'α-L-arabinofuranosidase B provenant de *Aspergillus niger.*

6. Procédé selon les revendications 1 à 5, dans lequel le moût est ensuite fermenté pour produire de la bière et/ou de l'alcool de consommation.

7. Utilisation d'α-L-arabinofuranosidase B provenant de *Aspergillus niger* dans un procédé pour améliorer la filtrabilité du moût.

8. Utilisation d'α-L-arabinofuranosidase A dans un procédé pour augmenter le rendement du moût.

9. Préparation d'enzyme appropriée pour une utilisation dans un procédé pour produire du moût, ladite composition comprenant un mélange d'activités enzymatiques choisi parmi un mélange comprenant au moins une activité de β-glucanase et une activité de libération d'α-L-arabinofuranose ou un mélange comprenant au moins une activité de β-glucanase, une activité d'endoxylanase et une activité de libération d'α-L-arabinofuranose.

10. Préparation d'enzyme selon la revendication 9, dans laquelle ladite activité de libération d'α-L-arabinofuranose est assurée par une enzyme choisie parmi l'α-L-arabinofuranosidase (EC 3.2.1.55) et la (1->4)-β-D-arabinoxylan-arabinofuranohydrolase (AXH), ou un de leurs mélanges.

11. Préparation d'enzyme selon la revendication 10, dans laquelle ladite α-L-arabinofuranosidase ou ladite (1->4)-β-D-arabinoxylan-arabinofuranohydrolase (AXH) peut être obtenue à partir d'une souche de *Aspergillus.*
